# EUROPEAN PATENT APPLICATION

(11) **EP 4 582 097 A1**
(43) Date of publication of application: **09.07.2025**
(21) Application number: 23876375.9
(22) Date of filing: 30.08.2023
(51) Int. Cl.: A61K 39/00, A61K 39/08, A61K 39/10, A61K 39/05, A61K 39/39

(54) **DIPHTHERIA-TETANUS-PERTUSSIS COMPOUND ADJUVANT COMBINED VACCINE**

(30) Priority: 12.10.2022 CN 202211246369
(71) Applicant: Changchun Bcht Biotechnology Co., Jilin 130012 (CN)
(72) Inventor: WANG, Mengshu, Changchun, Jilin 130012 (CN); YANG, Jun, Changchun, Jilin 130012 (CN); JIANG, Tao, Changchun, Jilin 130012 (CN); WU, Yanchun, Changchun, Jilin 130012 (CN); YAN, Kunming, Changchun, Jilin 130012 (CN)
(74) Representative: Gill Jennings & Every LLP
(86) International application number: PCT/CN2023/115711
(87) International publication number: WO 2024/078170

(57) **Abstract**

Disclosed in the present invention is a compound adjuvant combined vaccine, comprising an immunogenic composition and a compound adjuvant. The immunogenic composition comprises a pertussis antigen, a diphtheria antigen and a tetanus antigen; and the compound adjuvant is composed of an aluminum adjuvant and a TLR9 receptor agonist. Further disclosed in the present invention is a use of the compound adjuvant in the preparation of the compound adjuvant combined vaccine for preventing pertussis, diphtheria and tetanus in a subject.

## Description

### Technical Field

The present invention relates to the field of biological products, especially a compound adjuvant combined vaccine containing antigens of pertussis, diphtheria, and tetanus.

### Background Art

Pertussis is a severe acute respiratory tract infectious disease caused by *Bordetella pertussis.* It can affect people of any age, but infants and young children under the age of five are more susceptible. This disease is transmitted through droplets with high infectivity. The typical clinical symptom of pertussis is persistent paroxysmal cough ending in an inspiratory whoop or accompanied by vomiting, which tends to be complicated by pneumonia and encephalitis as the main causes of death. In developed countries, the lethality rate of pertussis is 0.1%, while in developing countries, the average lethality rates for infants and 1- to 4-year-old children are 3.9% and 1% respectively. The World Health Organization (WHO) estimates that there are approximately 60 million new cases of pertussis globally each year including 0.5-1.0 million deaths, seriously endangering the lives and health of children.

Diphtheria is an acute upper respiratory tract infectious disease caused by gram-positive *Corynebacterium diphtheriae.* It is clinically manifested by upper respiratory tract inflammation, typically in the pharynx, and sometimes in the postnasal space, larynx, and trachea; and can even cause damage to other organs, mainly cardiac muscle and peripheral nerves. The highly pathogenic exotoxin produced by toxigenic strains of *Corynebacterium diphtheriae* can form a local membrane and cause systemic organ damage as well. Clinical characteristics of diphtheria are pseudomembrane formation in the pharynx, larynx, nose, etc. and systemic toxic symptoms such as fever, fatigue, nausea and vomiting, and headache, which can be complicated by myocarditis and neuroparalysis in severe cases. Diphtheria was the main cause of death for children in the United Kingdom in the early 20th century. Since the introduction of diphtheria toxoid vaccination, the incidence rate of diphtheria has decreased significantly, but it continues to occur in some other areas. It is estimated that before the implementation of the Expanded Programme on Immunization (EPI) of WHO, there were nearly one million cases of diphtheria annually in third world countries, including 50,000 to 60,000 deaths.

Tetanus, as a disease endangering people's lives and health seriously, is an acute specific infection that can be caused by *Clostridium tetani* invading a wound of the human body, growing and reproducing, and producing toxins. *Clostridium tetani* and its toxins cannot invade normal skin or mucosa, and therefore tetanus typically occurs after an injury. All open injuries provide the possibility of tetanus. Tetanus is an acute specific infection caused by *Clostridium tetani* reproducing after invasion into a wound and secreting toxins, and manifested mainly by persistent contractions and paroxysmal spasms of systemic or local muscle. It is even more severe in impoverished and underdeveloped countries and regions. It is estimated that approximately one million cases occur globally each year, with the fatality rate around 50%. In developing countries, the tetanus fatality rate in newborns is as high as 90%.

Vaccine immunization is an effective means of preventing the above-mentioned diseases. The whole-cell pertussis vaccine has been used in countries all over the world for nearly 60 years since its development in the 1940s. The whole-cell pertussis vaccine has played a huge role in the prevention and control of pertussis, rendering it an infectious disease preventable with a vaccine, and it has been listed as one of the infectious diseases to be controlled and eliminated in the Expanded Programme on Immunization (EPI) initiated by the World Health Organization. Diphtheria toxoid can effectively prevent diphtheria, control the epidemic thereof, and ensure the physical health and life safety of children. At present, WHO and the Chinese government have listed diphtheria as one of the six infectious diseases specially controlled in EPI. Epidemiological studies have proven diphtheria toxoid to be a safe and effective vaccine against diphtheria epidemic. Tetanus toxoid can effectively prevent tetanus. Immunization with tetanus toxoid can induce an immune response in the body of 74-90% of the inoculated. The tetanus vaccine was launched in the United States of America in 1938, and was widely used in the military in 1941. Epidemiological studies have proven tetanus toxoid to be a safe and effective vaccine against tetanus epidemic. In 1948, the whole-cell diphtheria-tetanus-pertussis combined vaccine was launched and used in the United States of America. At the end of 1996, the diphtheria-tetanus-pertussis acellular component vaccine was approved for marketing.

It is stipulated in China's "Childhood Immunization Schedule of National Immunization Program Vaccines and Instructions (2021 Edition)" that one dose of Diphtheria, Tetanus and Acellular Pertussis Combined Vaccine, Adsorbed is given at 3 months, 4 months, 5 months, and 18 months of age respectively, and one dose of Diphtheria and Tetanus Combined Vaccine, Adsorbed is given at 6 years of age. The diphtheria-tetanus-pertussis vaccines currently marketed and used in China are co-purified vaccines mainly comprising two antigens, pertussis toxoid (PT) and filamentous hemagglutinin (FHA), whose production involves purifying pertussis antigens by the method of ammonium sulfate salting out. The diphtheria-tetanus-pertussis vaccines currently used in European and American countries are mainly component vaccines comprising three components, pertussis toxoid, filamentous hemagglutinin, and pertussis pertactin (PRN), in definite amounts, which are obtained by purification of pertussis antigens using the method of column chromatography. Component vaccines are advantageous in their definite components, high antigen purity, and few side effects. At present, studies in China on vaccines comprising PT, FHA, PRN, and antigen components of diphtheria and tetanus have entered the stage of clinical research.

With the widespread use of vaccines, the incidence rates of pertussis, diphtheria, and tetanus have decreased significantly. But in recent years, the incidence rate of pertussis in preschool children and adolescents has risen. Between 1922 and 2017 in European and American countries, pertussis continued to infect all age groups, with the number of cases increasing year by year. In 2005, the Advisory Committee on Immunization Practices (ACIP) recommended a one-off vaccination of adolescents and adults under 65 with diphtheria-tetanus-pertussis (acellular component) vaccine. In 2012, ACIP expanded the recommendation to include all adults, recommending that people over the age of 10 be given a diphtheria-tetanus-pertussis combined vaccine every 10 years. European and American countries have started controlling infection by booster immunization in adolescents and adults. However, there is yet to be a diphtheria-tetanus-pertussis three-component vaccine for adolescents and adults independently developed in China. Therefore, the development of a diphtheria-tetanus-pertussis vaccine for adolescents and adults in China is of great significance.

In all diphtheria-tetanus-pertussis combined vaccines currently marketed and used, aluminum adjuvants are used to enhance the immune response. Aluminum adjuvants can effectively induce a strong humoral immune response in the body, i.e. the Th2-type immune response, but they are less potent in inducing cellular immunity (the Th1-type immune response). CpG ODNs are synthetic ODNs containing unmethylated CpG motifs, which have a strong immunostimulatory effect. When used in combination with other adjuvants, they can significantly enhance specific humoral and cellular immune responses to antigens.

### Summary of the Invention

In view of the above, the object of the present invention is to provide a compound adjuvant combined vaccine comprising pertussis toxoid (PT), filamentous hemagglutinin (FHA), diphtheria toxoid (DT), and tetanus toxoid (TT) antigens, and further comprising a compound adjuvant such as a composition of an aluminum adjuvant and a TLR9 receptor agonist. The compound adjuvant combined vaccine may be used in preventing pertussis, diphtheria, and tetanus in infants and young children, adolescents, and adults.

In order to achieve the aforementioned object of the invention, the present invention provides the following technical solutions:
In the first aspect, the present invention provides an immunogenic composition comprising pertussis antigen, diphtheria antigen, and tetanus antigen. Preferably, it comprises pertussis PT antigen, pertussis FHA antigen, diphtheria DT antigen, and tetanus TT antigen.

In the second aspect, the present invention provides a compound adjuvant composed of an aluminum adjuvant and a TLR9 receptor agonist. Preferably, the aluminum adjuvant is aluminum hydroxide, and the TLR9 receptor agonist is a CpG ODN.

In the third aspect, the present invention provides a compound adjuvant combined vaccine comprising the immunogenic composition of the first aspect and the compound adjuvant of the second aspect and/or a pharmaceutically acceptable carrier. Preferably, the compound adjuvant is a composition of aluminum hydroxide and CpG ODN.

Preferably, each dose of the compound adjuvant combined vaccine contains: 1-25 µg of pertussis PT antigen, 1-25 µg of pertussis FHA antigen, 1-20 Lf of diphtheria DT antigen, and 1-5 Lf of tetanus TT antigen; more preferably, 4, 8, 16, or 25 µg of pertussis PT antigen, 4, 8, 16, or 25 µg of pertussis FHA antigen, 1, 2, 4, or 20 Lf of diphtheria DT antigen, and 1.5, 2.5, or 5 Lf of tetanus TT antigen; most preferably, 8 µg of pertussis PT antigen, 8 µg of pertussis FHA antigen, 2 Lf of diphtheria DT antigen, and 2.5 Lf of tetanus TT antigen.

Preferably, each dose of the compound adjuvant combined vaccine contains: 0.01-1 mg of aluminum and 0.1-100 µg of CpG ODN; more preferably, each dose of the compound adjuvant combined vaccine contains: 0.1-0.5 mg of aluminum and 25-100 µg of CpG ODN; most preferably, each dose of the compound adjuvant combined vaccine contains: 0.24 mg of aluminum and 50 µg or 100 µg of CpG ODN.

In the fourth aspect, the present invention provides use of the compound adjuvant of the second aspect in the manufacture of the compound adjuvant combined vaccine of the third aspect for preventing pertussis, diphtheria, and tetanus in a subject.

Preferably, the compound adjuvant combined vaccine comprises an immunogenic composition and a compound adjuvant.

Preferably, the subject is a mammal; more preferably, the subject is a human.

The compound adjuvant combined vaccine prepared by the present invention has the following advantages: by using an aluminum adjuvant and a TLR9 receptor agonist as a compound adjuvant, it can achieve higher immunogenicity with a lower amount of antigens used, and the potency of each component meets the relevant requirements in the Chinese Pharmacopoeia (2020 Edition). The present invention has strong immunogenicity and protective effect even in the absence of pertussis pertactin antigen, and may be used in preventing pertussis, diphtheria, and tetanus in infants and young children, adolescents, and adults.

### Detailed Description of the Invention

The present invention provides a compound adjuvant combined vaccine comprising pertussis PT antigen, pertussis FHA antigen, diphtheria DT antigen, and tetanus TT antigen, and further comprising a compound adjuvant composed of an aluminum adjuvant and a TLR9 receptor agonist. The compound adjuvant combined vaccine may prevent pertussis, diphtheria, and tetanus in infants and young children, adolescents, and adults.

The present invention is further detailed with reference to the following examples, without being limited thereto:

### Example 1: Exploration of adjuvant amounts in the compound adjuvant combined vaccine

1) The pertussis (PT antigen and FHA antigen) stock solution (prepared according to Examples 1, 2, 4, and 5 of the patent with Publication No. CN103242434B), purified diphtheria toxoid stock solution (with respect to the fermentation, reference is made to Cox, J C. New method for the large-scale preparation of diphtheria toxoid: purification of toxin [J]. Applied Microbiology, 1975, 29(4): 464-468; with respect to the preparation and purification, reference is made to Robb L A, Stainer D W, Scholte M J, Preparation and properties of diphtheria toxoids in submerged culture. IV. A revised method for the purification of diphtheria toxin [J]. Can J Microbiol, 1970, 16: 639; with respect to the preparation and detoxification, reference is made to Xiao Xiling, Shi Cuifeng, Ma Xueyan, et al. Irreversible toxoiding of the purified diphtheria toxin [J]. Chinese Journal of Biologicals, 1990, 1(3): 26-29), and purified tetanus toxoid stock solution (with respect to the fermentation and detoxification, reference is made to Lo C. H. Experiments on the production of tetanus toxin and toxoid [J]. Acta Microbiologica Sinica, 1953, 1(1): 113-126; with respect to the preparation and purification, reference is made to Stojićević Ivana, Dimitrijević Ljiljana, Dovezenski Nebojša et al. Tetanus toxoid purification: chromatographic procedures as an alternative to ammonium-sulphate precipitation [J]. J Chromatogr B Analyt Technol Biomed Life Sci, 2011, 879: 2213-2219) were individually mixed with and adsorbed onto aluminum hydroxide at given ratios, and the pH was adjusted to 6.5. Then CpG ODN 2006 adjuvant was added and well mixed.
2) The components adsorbed were mixed, so that each 0.5 mL dose contained 8 µg of pertussis PT antigen, 8 µg of pertussis FHA antigen, 2 Lf of diphtheria DT antigen, 2.5 Lf of tetanus TT antigen, 0.24 mg of aluminum, and 25, 50, or 100 µg of CpG ODN 2006. The specific groups were 25 µg CpG ODN 2006 group (Group 1), 50 µg CpG ODN 2006 group (Group 2), and 100 µg CpG ODN 2006 group (Group 3).
3) Meanwhile, a diphtheria-tetanus-pertussis vaccine group containing Al adjuvant alone (Group 4), a diphtheria-tetanus-pertussis vaccine group containing CpG-ODN-2006 adjuvant alone (Group 5), an aluminum hydroxide control group (Group 6), a CpG ODN 2006 control group (Group 7), and an antigen control group (Group 8) were prepared. The composition and amounts of antigen components and adjuvants in this example are shown in Table 1.

**Table 1 Composition and amounts of antigens and adjuvants**

| | PT (µg/dose) | FHA (µg/dose) | DT (Lf/dose) | TT (Lf/dose) | Al (mg/dose) | CpG ODN 2006 (µg/dose) |
|---|---|---|---|---|---|---|
| Group 1 | 8 | 8 | 2 | 2.5 | 0.24 | 25 |
| Group 2 | 8 | 8 | 2 | 2.5 | 0.24 | 50 |
| Group 3 | 8 | 8 | 2 | 2.5 | 0.24 | 100 |
| Group 4 | 8 | 8 | 2 | 2.5 | 0.24 | 0 |
| Group 5 | 8 | 8 | 2 | 2.5 | 0 | 50 |
| Group 6 | 0 | 0 | 0 | 0 | 0.24 | 0 |
| Group 7 | 0 | 0 | 0 | 0 | 0 | 100 |
| Group 8 | 8 | 8 | 2 | 2.5 | 0 | 0 |

### Example 2: Study on the effect of adjuvant amounts in the compound adjuvant combined vaccine on protective potency

1) Pertussis potency: the test was carried out according to the Chinese Pharmacopoeia (2020 Edition). The national potency standard (provided by China's National Institutes for Food and Drug Control) was diluted to 1, 0.2, and 0.04 IU/mL, and test animals were immunized with the diluted national potency standards, and the compound adjuvant combined vaccines prepared according to Table 1 respectively. The test animals, clean NIH mice, were injected intraperitoneally with 0.5 mL per mouse, challenged 21 days after the immunization, and observed for 14 days for survival after the challenge, and the potency was calculated.
2) Diphtheria potency: the test was carried out according to the Chinese Pharmacopoeia (2020 Edition). The national potency standard (provided by China's National Institutes for Food and Drug Control) was diluted to 5.54, 2.77, 1.38, and 0.69 IU/mL, and test animals were immunized with the diluted national potency standards, and the compound adjuvant combined vaccines prepared according to Table 1 respectively. The test animals, clean NIH mice, were injected subcutaneously with 0.5 mL per mouse, and blood was collected 5 weeks after the immunization. The serum was separated and tested by a toxin neutralization test using Vero cells, and the diphtheria potency was calculated from the cell survival.
3) Tetanus potency: the test was carried out according to the Chinese Pharmacopoeia (2020 Edition). The national potency standard (provided by China's National Institutes for Food and Drug Control) was diluted to 9.64, 4.82, 2.41, and 1.205 IU/mL, and test animals were immunized with the diluted national potency standards, and the compound adjuvant combined vaccines prepared according to Table 1 respectively. The test animals, clean NIH mice, were injected subcutaneously with 0.5 mL per mouse, challenged 4 weeks after the immunization, and observed for 5 days for survival after the challenge, and the potency was calculated.
4) The protective potency of each component is shown in Table 2.

**Table 2 Potency test results for each group**

| | Pertussis (IU/dose) | Diphtheria (IU/dose) | Tetanus (IU/dose) |
|---|---|---|---|
| Group 1 | 4.1 | 32 | 41.9 |
| Group 2 | 4.4 | 32.8 | 49.3 |
| Group 3 | 5.6 | 32 | 53.7 |
| Group 4 | 1.8 | 6.3 | 8.2 |
| Group 5 | 1.9 | 4.4 | 4.7 |
| Group 6 | 0 | 0 | 0 |
| Group 7 | 0 | 0 | 0 |
| Group 8 | 0 | 0 | 0 |

### Example 3: Exploration of antigen amounts in the compound adjuvant combined vaccine

1) The pertussis (PT antigen and FHA antigen) stock solution (prepared according to Examples 1, 2, 4, and 5 of the patent with Patent Publication No. CN103242434B), purified diphtheria toxoid stock solution (with respect to the fermentation, reference is made to Cox, J C. New method for the large-scale preparation of diphtheria toxoid: purification of toxin [J]. Applied Microbiology, 1975, 29(4): 464-468; with respect to the preparation and purification, reference is made to Robb L A, Stainer D W, Scholte M J, Preparation and properties of diphtheria toxoids in submerged culture. IV. A revised method for the purification of diphtheria toxin [J]. Can J Microbiol, 1970, 16: 639; with respect to the preparation and detoxification, reference is made to Xiao Xiling, Shi Cuifeng, Ma Xueyan, et al. Irreversible toxoiding of the purified diphtheria toxin [J]. Chinese Journal of Biologicals, 1990, 1(3): 26-29), and purified tetanus toxoid stock solution (with respect to the fermentation and detoxification, reference is made to Lo C. H. Experiments on the production of tetanus toxin and toxoid [J]. Acta Microbiologica Sinica, 1953, 1(1): 113-126; with respect to the preparation and purification, reference is made to Stojićević Ivana, Dimitrijević Ljiljana, Dovezenski Nebojša et al. Tetanus toxoid purification: chromatographic procedures as an alternative to ammonium-sulphate precipitation [J]. J Chromatogr B Analyt Technol Biomed Life Sci, 2011, 879: 2213-2219) were individually mixed with and adsorbed onto aluminum hydroxide at given ratios, and the pH was adjusted to 6.5. Then CpG ODN 2006 adjuvant was added and well mixed.
2) The components adsorbed were mixed, so that each dose contained 4, 8, 16, or 25 µg of pertussis PT antigen, 4, 8, 16, or 25 µg of pertussis FHA antigen, 1, 2, 4, or 20 Lf of diphtheria DT antigen, 1.5, 2.5, or 5 Lf of tetanus TT antigen, 0.24 mg of aluminum, and 50 or 0 µg of CpG ODN 2006. The groups were Group 1, Group 2, Group 3, Group 4, and Group 5, and the details of each component are shown in Table 3.

The composition and amounts of antigen components and adjuvants in this example are shown in Table 3.

**Table 3 Composition and amounts of antigens and adjuvants**

| | PT (µg/dose) | FHA (µg/dose) | DT (Lf/dose) | TT (Lf/dose) | Al (mg/dose) | CpG ODN 2006 (µg/dose) |
|---|---|---|---|---|---|---|
| Group 1 | 4 | 4 | 1 | 1.5 | 0.24 | 50 |
| Group 2 | 8 | 8 | 2 | 2.5 | 0.24 | 50 |
| Group 3 | 16 | 16 | 4 | 5 | 0.24 | 50 |
| Group 4 | 25 | 25 | 20 | 5 | 0.24 | 50 |
| Group 5 | 25 | 25 | 20 | 5 | 0.24 | 0 |

### Example 4: Study on the effect of antigen amounts in the compound adjuvant combined vaccine on protective potency

1) Pertussis potency: the test was carried out according to the Chinese Pharmacopoeia (2020 Edition). The national potency standard (provided by China's National Institutes for Food and Drug Control) was diluted to 1, 0.2, and 0.04 IU/mL, and test animals were immunized with the diluted national potency standards, the compound adjuvant combined vaccines prepared according to Table 3 and a commercially available vaccine (diphtheria, tetanus, pertussis (acellular component), poliomyelitis (inactivated) vaccine and *Haemophilus influenzae* type b conjugate vaccine, adsorbed, available from Sanofi Pasteur S.A.) respectively. The test animals, clean NIH mice, were injected intraperitoneally with 0.5 mL per mouse, challenged 21 days after the immunization, and observed for 14 days for survival after the challenge, and the potency was calculated.
2) Diphtheria potency: the test was carried out according to the Chinese Pharmacopoeia (2020 Edition). The national potency standard (provided by China's National Institutes for Food and Drug Control) was diluted to 5.54, 2.77, 1.38, and 0.69 IU/mL, and test animals were immunized with the diluted national potency standards, the compound adjuvant combined vaccines prepared according to Table 3 and a commercially available vaccine (diphtheria, tetanus, pertussis (acellular component), poliomyelitis (inactivated) vaccine and *Haemophilus influenzae* type b conjugate vaccine, adsorbed, available from Sanofi Pasteur S.A.) respectively. The test animals, clean NIH mice, were injected subcutaneously with 0.5 mL per mouse, and blood was collected 5 weeks after the immunization. The serum was separated and tested by a toxin neutralization test using Vero cells, and the diphtheria potency was calculated from the cell survival.
3) Tetanus potency: the test was carried out according to the Chinese Pharmacopoeia (2020 Edition). The national potency standard (provided by China's National Institutes for Food and Drug Control) was diluted to 9.64, 4.82, 2.41, and 1.205 IU/mL, and test animals were immunized with the diluted national potency standards, the compound adjuvant combined vaccines prepared according to Table 3 and a commercially available vaccine (diphtheria, tetanus, pertussis (acellular component), poliomyelitis (inactivated) vaccine and *Haemophilus influenzae* type b conjugate vaccine, adsorbed, available from Sanofi Pasteur S.A.) respectively. The test animals, clean NIH mice, were injected subcutaneously with 0.5 mL per mouse, challenged 4 weeks after the immunization, and observed for 5 days for survival after the challenge, and the potency was calculated.
4) The potency results of each component are shown in Table 4.

**Table 4 Protective potency results for each group**

| | Pertussis (IU/dose) | Diphtheria (IU/dose) | Tetanus (IU/dose) |
|---|---|---|---|
| Group 1 | 3.8 | 24.5 | 41.7 |
| Group 2 | 4.5 | 33.2 | 50.8 |
| Group 3 | 8.6 | 40.4 | 72.1 |
| Group 4 | 10.2 | 63.7 | 97.5 |
| Group 5 | 4.7 | 37.1 | 48.2 |
| Commercially available vaccine | 4.8 | 33.7 | 44.1 |
| (diphtheria, tetanus, pertussis (acellular component), poliomyelitis (inactivated) vaccine and *Haemophilus influenzae* type b conjugate vaccine, adsorbed, available from Sanofi Pasteur S.A.) | | | |

The experimental results above indicate the following:
As shown by Examples 1 and 2, under the same antigen and aluminum adjuvant contents, the potency of each antigen (except diphtheria antigen) exhibits dosage effect with the increase in the CpG ODN 2006 content (i.e. the potency of each antigen (except diphtheria antigen) increases with the CpG ODN 2006 content); and the compound adjuvant has a synergistic effect (i.e. the potencies of the groups using the compound adjuvant (Groups 1, 2, and 3) are all higher than the sum of the potencies of the single-adjuvant groups (Group 4 + Group 5)). The potency of each compound adjuvant group meets the standards in the Chinese Pharmacopoeia (2020 Edition); i.e. each single human dose of 0.5 mL should contain not less than 4.0 IU of acellular pertussis vaccine, not less than 30 IU of diphtheria toxoid and not less than 40 IU of tetanus toxoid.

As shown by Examples 3 and 4, the use of the diphtheria-tetanus-pertussis vaccine containing compound adjuvant can effectively reduce the amount of antigens used. Group 2 contains 8 µg each of PT and FHA, while the commercially available vaccine group contains 25 µg each of PT and FHA, but their pertussis potencies are almost equivalent. When Group 3 contains 16 µg each of PT and FHA, its pertussis potency is significantly higher than that of the commercially available vaccine group. Since the definitions of diphtheria and tetanus antigen contents in the specification of the commercially available vaccine are different from those used in China, no further comparison is made here.

As shown by Examples 1, 2, 3, and 4, when the concentration of CpG ODN 2006 in the compound adjuvant is increased to 50-100 µg/dose, the pertussis potency is equal to or significantly higher than that of the commercially available vaccine group.

Therefore, the present invention enables the potencies of pertussis, diphtheria, and tetanus to increase with their amounts in the absence of pertussis pertactin antigen by using an aluminum hydroxide-CpG ODN 2006 compound adjuvant. In comparison with the use of an aluminum adjuvant or CpG ODN 2006 adjuvant alone, it may reduce antigen amounts while maintaining comparable potencies, and may be used in preventing pertussis, diphtheria, and tetanus in infants and young children, adolescents, and adults.

## Claims

1. A compound adjuvant combined vaccine, comprising an immunogenic composition and a compound adjuvant and/or a pharmaceutically acceptable carrier, wherein the immunogenic composition comprises pertussis PT antigen, pertussis FHA antigen, diphtheria DT antigen, and tetanus TT antigen, and wherein the compound adjuvant is composed of an aluminum adjuvant and a TLR9 receptor agonist.

2. The compound adjuvant combined vaccine according to claim 1, wherein each dose of the compound adjuvant combined vaccine contains 1-25 µg of the pertussis PT antigen, 1-25 µg of the pertussis FHA antigen, 1-20 Lf of the diphtheria DT antigen, and 1-5 Lf of the tetanus TT antigen.

3. The compound adjuvant combined vaccine according to claim 2, wherein each dose of the compound adjuvant combined vaccine contains 4, 8, 16, or 25 µg of the pertussis PT antigen, 4, 8, 16, or 25 µg of the pertussis FHA antigen, 1, 2, 4, or 20 Lf of the diphtheria DT antigen, and 1.5, 2.5, or 5 Lf of the tetanus TT antigen.

4. The compound adjuvant combined vaccine according to claim 3, wherein each dose of the compound adjuvant combined vaccine contains 8 µg of the pertussis PT antigen, 8 µg of the pertussis FHA antigen, 2 Lf of the diphtheria DT antigen, and 2.5 Lf of the tetanus TT antigen.

5. The compound adjuvant combined vaccine according to any one of claims 1-4, wherein the aluminum adjuvant is aluminum hydroxide, and the TLR9 receptor agonist is a CpG ODN.

6. The compound adjuvant combined vaccine according to claim 5, wherein the CpG ODN is CpG ODN 2006.

7. The compound adjuvant combined vaccine according to claim 5, wherein each dose of the compound adjuvant combined vaccine contains 0.01-1 mg of aluminum and 0.1-100 µg of CpG ODN.

8. The compound adjuvant combined vaccine according to claim 7, wherein each dose of the compound adjuvant combined vaccine contains 0.1-0.5 mg of aluminum and 25-100 µg of CpG ODN; preferably, each dose of the compound adjuvant combined vaccine contains 0.24 mg of aluminum and 50 µg or 100 µg of CpG ODN.

9. Use of a compound adjuvant in the manufacture of the compound adjuvant combined vaccine according to any one of claims 1-8 for preventing pertussis, diphtheria, and tetanus in a subject, wherein the compound adjuvant is composed of an aluminum adjuvant and a TLR9 receptor agonist.

10. The use according to claim 9, wherein the subject is a mammal; more preferably, the subject is a human.
